# EUROPEAN PATENT SPECIFICATION

(11) **EP 3 538 177 B1**
(45) Date of publication and mention of the grant of the patent: **02.09.2020**
(21) Application number: 17805275.9
(22) Date of filing: 08.11.2017
(51) Int. Cl.: A61M 1/36, A61B 5/1455

(54) **DEVICE FOR OPTICAL MEASUREMENT**
VORRICHTUNG ZUR OPTISCHEN MESSUNG
DISPOSITIF DE MESURE OPTIQUE

(30) Priority: 09.11.2016 IT 201600112827
(43) Date of publication of application: 18.09.2019
(73) Proprietor: Allmed Medical Care Holdings Limited, London W4 5YS (GB)
(72) Inventor: DELNEVO, Annalisa, London W4 5YS (GB)
(74) Representative: Zoli, Filippo
(86) International application number: PCT/IB2017/056973
(87) International publication number: WO 2018/087664

(56) References cited:
- EP-A2- 2 227 270
- WO-A1-98/37801
- US-A1- 2010 110 416

## Description

### Technical Field

The present invention refers to a device for optical measurement, particularly for extracorporeal blood circuits.

### Background Art

Extracorporeal blood circuits are used to move blood outside the body and includes a venous line and an arterial line connecting the patient to a blood treatment unit, for instance a dialyzer, an hemofilter, a plasmafilter, etc .... During the extracorporeal blood circulation it can be necessary to perform the measurement of some blood values like, for instance, haemoglobin concentration.

The haemoglobin concentration can be measured by means of intrusive measurements, which require the laboratory examination of blood samples, or by means of non-intrusive measurements made within the machine which performs the extracorporeal circulation. This kind of measurements has the advantage of being provided in real time, so allowing the correction of the operating parameters substantially instantaneously.

In order to perform these measurements, the extracorporeal blood circuit is generally provided with a tubular body, both rigid and transparent, rigid piece of transparent tube, which is engaged, for example, at the outlet of a chamber placed along the arterial line and which is designed to be received in an appropriate holder on the machine which performs the extracorporeal circulation. An emitter and a sensor are applied to the machine for emitting and then detecting the absorption of electromagnetic waves subsequent to the detection of the presence of the tubular body.

Some examples of methods for measuring the haemoglobin concentration in an extracorporeal blood circuits of a dialysis machine are known by US 6794194, EP 0467805 and EP 2227270.

More in particular, US 6794194 discloses the use of a rigid piece of transparent tube including a pressure transducer applied in correspondence of the arterial line in a position where an electromagnetic wave sensor and a pressure sensor are both supported by the machine.

EP 0467805 shows a blood treatment apparatus having an optical/electronic system comprising a LED diode and a photosensitive sensor capable of receiving the light radiation emitted by the LED and of providing a corresponding electrical signal. Furthermore it provides a circuit for processing this electrical signal so as to discriminate when, in use, the tubular body through which blood flows is placed between LED diode and sensor.

Finally, EP 2227270 discloses an apparatus for the extracorporeal treatment of blood comprising a rigid tubular piece having a protruding element, which is in one piece with the rigid tubular piece and which is designed to cooperate, in use, with a corresponding mating recess provided on a holder of the medical apparatus for activating the measurement. The protruding element disclosed by EP 2227270 comprises side walls emerging from an external surface of the end portion of the tubular body and a terminal wall extending transversally to said side walls.

The apparatus disclosed by EP 2227270, which has been developed for improving the devices disclosed by US 6794194 and EP 0467805, has a number of drawbacks.

In particular the rigid tubular body has to be molded in one piece and, in order to allow the optical sensor to function properly, the tolerances have to be very tight, even if the protruding element as such doesn't require tight tolerances for exploiting its function. As a consequence, the complexity of the mold for producing the tubular body and the related cost are very high.

In addition, whenever the optical measurements are implemented at different light frequencies, it is necessary to manufacture a corresponding number of molds, so as to obtain, for each light frequency used, a tubular body for the optical measurement and the corresponding protruding element for activating the switch.

Another drawback related to the apparatus disclosed by EP 2227270 is that the rigid tubular piece has to be positioned in a very precise way onto the relative machine, otherwise the protruding element cannot be introduced into the corresponding mating recess and the optical measurement cannot be authorized. Moreover, the apparatus disclosed by EP 2227270 needs to be assembled to the relative tubes with a very precise orientation, otherwise, the tubes can be twisted together as a consequence of the coupling to the machine and this would risk to compromise the safety of the treatment.

A further drawback consists in the difficulty of connecting the device to the holder, due to the fact that the tubular piece, the protruding element and the holder itself are of the rigid type.

WO 98/37801 discloses a device for optical measurement comprising a plurality of separated portions, of which a first tubular portion and a second portion supporting the protruding element for activating the measurement, where the second portion is suitable to cooperate with the first portion for fixing the position of the protruding element with respect to it.

### Disclosure of the Invention

The main aim of the present invention is to provide a device for optical measurement which requires less tight tolerances for the part suitable for activating the measurement.

Within this aim, one object of the present invention is to allow the alignment and the introduction of the protruding element within the corresponding mating recess with respect to the known devices.

One object of the present invention is to provide a device which is easier to be coupled to the relative holder with respect to the known devices.

A further object of the present invention is to provide a device where the part suitable for activating the measurement can be easily and cheaply replaced, while the tubular body remains the same.

Another object of the present invention is to provide a device for optical measurements which allows to overcome the mentioned drawbacks of the prior art within the ambit of a simple, rational, easy and effective use as well as low cost solution.

The above mentioned objects are achieved by the present device for optical measurements of the blood of a patient according to claim 1.

### Brief Description of the Drawings

Other characteristics and advantages of the present invention will become better evident from the description of a preferred but not exclusive embodiment of a device for optical measurements, illustrated by way of an indicative, but not limitative example in the accompanying drawings in which:
Figure 1 is a longitudinal cut-out graphic sectional view of a device according to the invention in a particular form of embodiment;
Figure 2 is a longitudinal sectional view of the assembled device of figure 1;
Figure 3 is a longitudinal sectional view of a device according to the invention in alternative form of embodiment;
Figure 4 is an axonometric view of the second portion of the device represented in figure 3;
Figure 5 is a longitudinal sectional view of a device according to the invention in a further form of embodiment;
Figure 6 is an axonometric view of the second portion of the device represented in figure 5;
Figure 7 is a longitudinal sectional view of a device according to the invention in a further form of embodiment;
Figure 8 represents a bottom plant views of the first tubular portion, in two particular form of embodiments.

### Ways of carrying out the Invention

With particular reference to these illustrations, a device for optical measurement, particularly for extracorporeal blood circuits, is globally indicated by reference number 1.

The device 1 comprises at least a tubular element 2 having a first end connector 3 and a second end connector 4, which are suitable to be connected to a first tube 5 and a second tube 6, respectively, for subjecting a fluid, for example blood, through the tubular element itself.

More in particular, the first end connector 3 and the second end connector 4 are opposite the one another and are arranged at the longitudinal extremities of the tubular element 2, which further comprises an intermediate portion 7 extending between the first end connector 3 and the second end connector 4.

The intermediate portion 7 is designed and calibrated for allowing, during the use, i.e. when blood or other work fluid flow along it, the non-invasive measurement of certain parameters of the fluid itself.

Preferably, the intermediate portion 7 has a constant cross section and is made of at least partially transparent material.

The cross section of the intermediate portion 7 may also be variable without compromising the measurement.

In term of shape the intermediate portion 7 may have toric cross section.

The tubular element 2 is of the rigid type and may be made, for example, of PETG (Polyethylene Terephthalate - Glycol); the use of other rigid materials of the known type cannot however be ruled out.

According to the invention, the tubular element 2 comprises at least two pieces, separated the one from the other, of which a first tubular portion 2a and at least a second portion 2b bearing a protruding element 8 suitable to be inserted into a corresponding mating recess provided on a medical apparatus (not represented in the figures) for activating the optical measurement, and where the second portion 2b is suitable to cooperate with the first tubular portion 2a for fixing the position of the protruding element 8 with respect to the first tubular portion itself.

The protruding element 8 may have a numbers of possible shapes different the one form the others, for instance its external surface may be curvilinear without interruption, or it may have a parallelepiped shape. In the preferred form of embodiments represented in the figures, the protruding element 8 is convex and rounded.

Conveniently, the first tubular portion 2a defines the intermediate portion 7.

In a first form of embodiment according to the invention, the protruding element 8 is made with yielding material, for instance soft PVC (Polyvinyl Chloride) LDPE (Low Density Polyethylene) or PP (Polypropylene). More in particular, the protruding element 8 is made with soft material.

The second portion 2b may be entirely made with yielding material or, alternatively, may comprise a base element made with a cheap material and easy to be manufactured, not necessary of the soft type, for instance PP (Polypropylene, POM (Polyoxymethylene), PVC (Polyvinyl Chloride), HDPE/LDPE (High Density/Low Density Polyethylene), and a covering element overlapped to the base element, defining the protruding element 8 and realized with soft material.

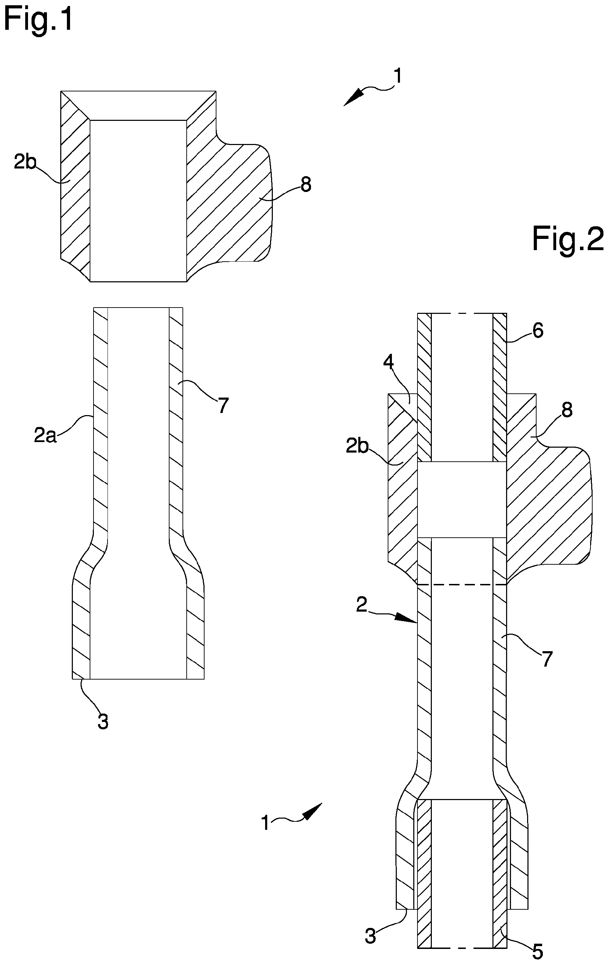

According to the form of embodiment represented in figures 1 and 2, the first tubular portion 2a and the second portion 2b define the first end connector 3 and the second end connector 4, respectively; in this second form of embodiment the second portion 2b has a tubular shape too. Furthermore, the second portion 2b is fitted on the first tubular portion 2a and is fixed to the same, for example by gluing, so as to obtain the tubular element 2.

In a second form of embodiment according to the invention, represented in the figures from 3 to 4, the first tubular portion 2a defines both the first end connector 3 and the second end connector 4 and the second portion 2b is movable with respect to the first tubular portion itself and is applied over the second tube 6 after its fitting onto the second end connector 4. In other words, the second portion 2b is secured to the first tubular portion 2a in the desired position by interposing the second tube 6 between them.

More in particular, the second portion 2b is movable in translation with respect to the first tubular portion 2a (and therefore with respect to the second tube 6) along a direction which is substantially parallel to its longitudinal axis (of the first tubular portion 2a), so as to adjust its height with respect to the corresponding holder, and/or is movable in rotation around the same longitudinal axis, so as to adjust its angular position with respect to the first tubular portion 2a (and therefore with respect to the second tube 6). Advantageously, the second portion 2b comprises means for securing 9 the second portion itself to the first tubular portion 2a.

The means for securing 9 are connected in a sliding and/or in a rotating manner to the first portion 2a by interposition of the second tube 6.

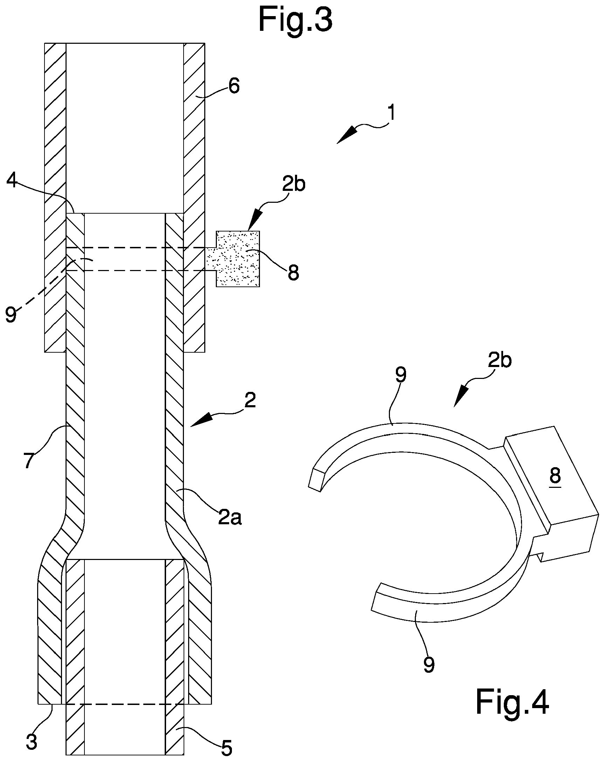

More in particular, the means for securing 9 allows to connect the second portion 2b to the first portion 2a, by interposition of the second tube 6, and, in the meantime, to change its position with respect to the first tubular portion itself so as to align the protruding element 8 to the corresponding mating recess. In the form of embodiment represented in figures 3 and 4, the second portion 2b is clip shaped and the means for securing 9 comprises two ribs which can be moved for applying the second portion itself to the second tube 6.

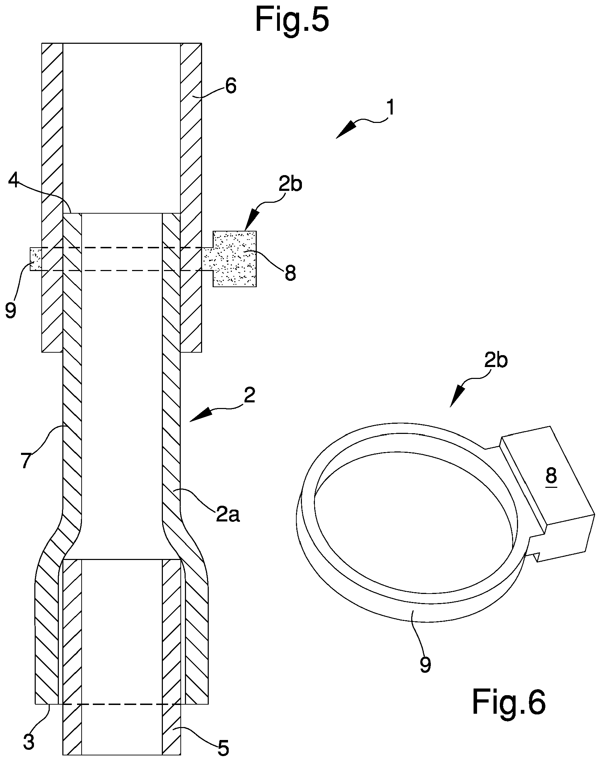

In the form of embodiment represented in figure 5 and 6, the means for securing 9 are annular shaped.

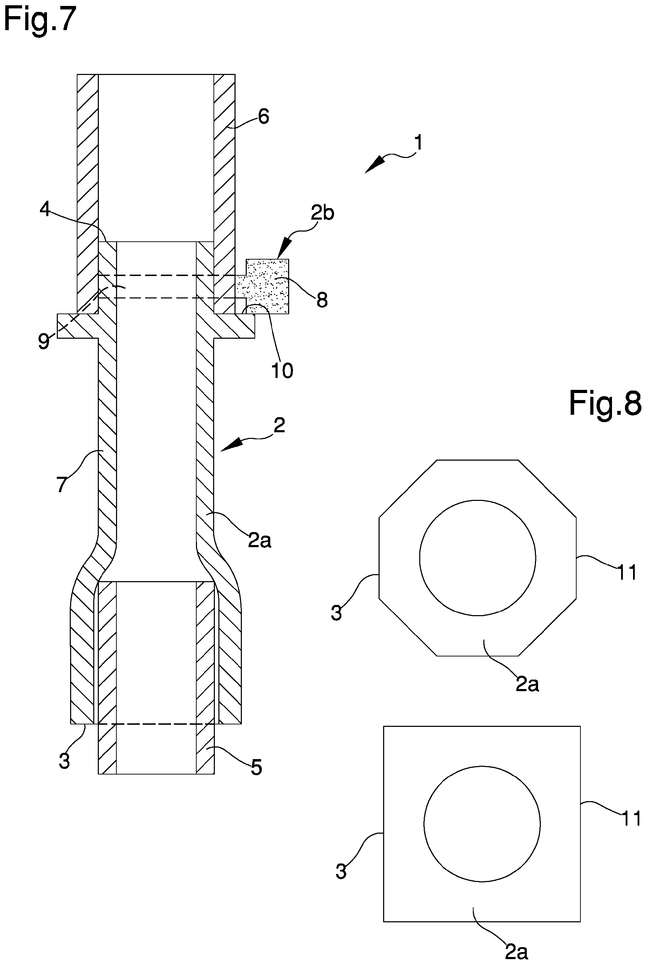

In a further form of embodiment, represented in figure 7, the first tubular portion 2a presents at least a stop surface 10, which protrudes radially outwardly, and suitable to define the end stroke position of the second portion 2b along the longitudinal direction. The stop surface 10 also defines a reference position for the second tube 6, whose extremity, in use, rests against the stop surface itself.

Advantageously, as represented in figure 8, the cross section of the first end connector 3 presents at least one external substantially flat surface 11, suitable to be arranged facing the dialysis machine, and suitable to define the angular position of the protruding element 8 with respect to the first tubular portion 2a, so as to allow an easier alignment of the second portion 2b with respect to the corresponding holder.

Preferably, the cross section of the first end connector 3 is polygonal.

The method for obtaining the device for optical measurement according to the invention is the following.

The method comprises the phases of furnishing at least a first tubular portion 2a and a second portion 2b, the one distinct from the other, and of connecting them to each other so as to obtain the tubular element 2.

As above disclosed, the protruding element 8 is realized separated by the first tubular portion 2a.

In the first form of embodiment according to the invention, the protruding element 8 is made with yielding material, preferably soft.

In a particular form of embodiment, the second portion 2b may be realized with the overmolding technique, i.e. by overlapping a base element realized with rigid material with a covering element realized with soft material.

In the form of embodiment represented in figures 1 and 2, the second portion 2b is secured to the first tubular portion 2a so as that the first tubular portion 2a and the second portion 2b define the first end connector 3 and the second end connector 4, respectively.

More in particular, the second portion 2b is fitted on the first tubular portion 2a, in correspondence of one of his longitudinal extremities.

The second portion 2b may be fixed with respect to the first tubular portion 2a by means of gluing or other known technique.

After having secured the second portion 2b to the first portion 2a, the end connectors 3, 4 are connected to the relative tubes 5, 6 and the protruding element 8 is inserted into the corresponding mating recess. The softness of the protruding element 8 allows to facilitate its insertion into the corresponding mating recess so as to compensate possible misalignment between them.

The method for realizing the devices relating to the second form of embodiments according to the invention, represented in the figures from 3 to 7, where the first tubular portion 2a defines both the end connectors 3, 4, comprises the phases of: connecting the first end connector 3 and the second end connector 4 to the relative tubes 5 and 6, applying the second portion 2b over the tube 6 fitted onto the first tubular portion 2a so as to secure it (the second portion 2b) with respect to both of them (the first tubular portion 2a and the relative tube), and moving the second portion 2b with respect to the first tubular portion 2a for aligning the protruding element 8 to the corresponding recess.

More in detail, the second portion 2b is applied to the first tubular portion 2a by embracing both the first tubular portion 2a and the tube 6 fitted on it.

It has in practice been ascertained how the described invention achieves the proposed objects and in particular the fact is underlined that the device according to the invention is very easy to be realized, so allowing to save costs and, in the meantime, to improve its usability with respect to the known devices.

More in particular, the fact of realizing two distinct portions allows to simplify the moulds; this is because the second portion bearing the protruding element may be realized with tolerances less tight with respect to the first tubular portion.

Moreover, this allows to optimize the warehouse stocks and, in the meantime, to change the shape of the protruding element for the coupling with the switch activating the optical measurement according to the necessities.

Furthermore, the realization of the protruding element with soft material allows to facilitate its insertion into the corresponding mating recess and to improve their reciprocal interaction.

Not the least, the alternative form of embodiment where the second portion is movable with respect to the first tubular portion allows to significantly simplify the phase of assembly as the first tubular portion can be fixed to the relative tubes in any position and the second portion can be moved at convenience of the operator so as to easily introduce the protruding element into the corresponding mating recess for activating the measurement. This further allows to avoid that the tubes are twisted together when the device is assembled to the machine.

## Claims

1. Device (1) for optical measurement, particularly for extracorporeal blood circuits, comprising at least a tubular element (2) having a first end connector (3) and a second end connector (4) connectable, respectively, to a first tube (5) and to a second tube (6) for subjecting a work fluid through the tubular element itself, and an intermediate portion (7) extending between said first end connector (3) and said second end connector (4) and suitable for subjecting the fluid to a non-invasive measurement of one or more parameters,
where said tubular element (2) comprises at least two pieces distinct the one another, of which a first tubular portion (2a) and at least a second portion (2b) bearing a protruding element (8) suitable to be inserted into a corresponding mating recess provided on a medical apparatus for activating the optical measurement, where said second portion (2b) is suitable to cooperate with said first tubular portion (2a) for fixing the position of said protruding element (8) with respect to the first tubular portion itself,
**characterized by** the fact that
said first tubular portion (2a) defines both said first and second end connectors (3, 4) and that said second portion (2b) is movable with respect to the first tubular portion itself and is applied over both the first tubular portion (2a) and the second tube (6) fitted on it;
or by the fact that
said first tubular portion (2a) is of the rigid type and that at least said protruding element (8) is realized with soft material.

2. Device (1) according to claim 1, **characterized by** the fact that said protruding element (8) is convex and rounded.

3. Device (1) according to claim 1 or 2, **characterized by** the fact that said second portion (2b) comprises at least a base element of the rigid type and at least a covering element applied over said base element, defining said protruding element (8) and realized with soft material.

4. Device (1) according to one or more of the preceding claims, **characterized by** the fact that said first tubular portion (2a) and said second portion (2b) define said first end connector (3) and said second end connector (4), respectively, said second portion (2b) being of the tubular type, and by the fact that said first tubular portion (2a) and said second portion (2b) are fixed to one another.

5. Device (1) according to one or more of the claims from 1 to 3, **characterized by** the fact that said second portion (2b) is movable in translation and/or in rotation with respect to said first tubular portion (2a) along and/or around, respectively, the longitudinal axis of the first tubular portion itself.

6. Device (1) according to claim 5, **characterized by** the fact that said second portion (2b) comprises anchoring means (9) of said second portion (2b) to said first tubular portion (2a), where said anchoring means (9) are connected in a sliding and/or in a rotating manner to said first tubular portion (2a) by interposition of the second tube (6).

7. Device (1) according to claim 5 or 6, **characterized by** the fact that said second portion (2b) is clip or annular shaped.

8. Device (1) according to one or more of the claims from 5 to 7, **characterized by** the fact that said first tubular portion (2a) presents at least a stop surface (10) defining the end stroke of said second portion (2b) during its movement along the longitudinal direction of the first tubular portion itself.

9. Device (1) according to claim 8, **characterized by** the fact that said stop surface (10) protrudes radially outwardly.

10. Device (1) according to one or more of the claims from 5 to 9, **characterized by** the fact that the cross section of said first end connector (3) presents at least an external flat surface (11) suitable to be placed, in use, faced to the medical apparatus and to define the angular position of said protruding element (8).

## Patentansprüche

1. Vorrichtung (1) zur optischen Messung, insbesondere für extrakorporale Blutkreisläufe, mit mindestens einem rohrförmigen Element (2), das ein erstes Endverbindungsstück (3) und ein zweites Endverbindungsstück (4) aufweist, die mit einem ersten Rohr (5) bzw. einem zweiten Rohr (6) verbindbar sind, um ein Arbeitsfluid durch das rohrförmige Element selbst auszusetzen, und mit einem Zwischenabschnitt (7), der sich zwischen dem ersten Endverbindungsstück (3) und dem zweiten Endverbindungsstück (4) erstreckt und geeignet ist, das Fluid einer nicht-invasiven Messung eines oder mehrerer Parameter auszusetzen,
wobei das rohrförmige Element (2) mindestens zwei voneinander verschiedene Teile umfasst, von denen ein erster rohrförmiger Abschnitt (2a) und mindestens ein zweiter Abschnitt (2b) ein vorstehendes Element (8) trägt, das geeignet ist, in eine entsprechende, passende Aussparung eingeführt zu werden, die an einem medizinischen Gerät zur Aktivierung der optischen Messung vorgesehen ist, wobei der zweite Abschnitt (2b) geeignet ist, mit dem ersten rohrförmigen Abschnitt (2a) zusammenzuwirken, um die Position des vorstehenden Elements (8) in Bezug auf den ersten rohrförmigen Abschnitt selbst zu fixieren,
**dadurch gekennzeichnet, dass**
der erste rohrförmige Abschnitt (2a) sowohl das erste als auch das zweite Endverbindungsstück (3, 4) definiert und dass der zweite Abschnitt (2b) in Bezug auf den ersten rohrförmigen Abschnitt selbst beweglich ist und sowohl über den ersten rohrförmigen Abschnitt (2a) als auch über das darauf angebrachte zweite Rohr (6) angebracht ist;
oder durch die Tatsache, dass
der erste rohrförmige Abschnitt (2a) vom starren Typ ist und dass zumindest das vorstehende Element (8) aus weichem Material realisiert ist.

2. Vorrichtung (1) nach Anspruch 1, **dadurch gekennzeichnet, dass** das vorstehende Element (8) konvex und abgerundet ist.

3. Vorrichtung (1) nach Anspruch 1 oder 2, **dadurch gekennzeichnet, dass** der zweite Abschnitt (2b) mindestens ein Basiselement vom starren Typ und mindestens ein über dem Basiselement angebrachtes Abdeckelement umfasst, das das vorstehende Element (8) definiert und aus weichem Material realisiert ist.

4. Vorrichtung (1) nach einem oder mehreren der vorhergehenden Ansprüche, **dadurch gekennzeichnet, dass** der erste rohrförmige Abschnitt (2a) und der zweite Abschnitt (2b) das erste Endverbindungsstück (3) beziehungsweise das zweite Endverbindungsstück (4) definieren, wobei der zweite Abschnitt (2b) vom rohrförmigen Typ ist, und durch die Tatsache, dass der erste rohrförmige Abschnitt (2a) und der zweite Abschnitt (2b) aneinander befestigt sind.

5. Vorrichtung (1) nach einem oder mehreren der Ansprüche von 1 bis 3, **dadurch gekennzeichnet, dass** der zweite Abschnitt (2b) in Translation und/oder in Rotation in Bezug auf den ersten rohrförmigen Abschnitt (2a) entlang und/oder entsprechend um die Längsachse des ersten rohrförmigen Abschnitts selbst beweglich ist.

6. Vorrichtung (1) nach Anspruch 5, **dadurch gekennzeichnet, dass** der zweite Abschnitt (2b) Verankerungsmittel (9) des zweiten Abschnitts (2b) an dem ersten rohrförmigen Abschnitt (2a) umfasst, wobei die Verankerungsmittel (9) gleitend und/oder drehend mit dem ersten rohrförmigen Abschnitt (2a) durch Einfügen des zweiten Rohrs (6) verbunden sind.

7. Vorrichtung (1) nach Anspruch 5 oder 6, **dadurch gekennzeichnet, dass** der zweite Abschnitt (2b) klammer- oder ringförmig ist.

8. Vorrichtung (1) nach einem oder mehreren der Ansprüche von 5 bis 7, **dadurch gekennzeichnet, dass** der erste rohrförmige Abschnitt (2a) mindestens eine Anschlagfläche (10) aufweist, die den Endhub des zweiten Abschnitts (2b) während seiner Bewegung entlang der Längsrichtung des ersten rohrförmigen Abschnitts selbst definiert.

9. Vorrichtung (1) nach Anspruch 8, **dadurch gekennzeichnet, dass** die Anschlagfläche (10) radial nach außen vorsteht.

10. Vorrichtung (1) gemäß einem oder mehreren der Ansprüche von 5 bis 9, **gekennzeichnet durch** die Tatsache, dass der Querschnitt des ersten Endverbindungsstücks (3) mindestens eine äußere flache Oberfläche (11) aufweist, die geeignet ist, im Gebrauch gegenüber dem medizinischen Gerät angeordnet zu werden und die Winkelposition des vorstehenden Elements (8) zu definieren.

## Revendications

1. Dispositif (1) de mesure optique, en particulier pour circuits sanguins extracorporels, comprenant au moins un élément tubulaire (2) ayant un premier raccord d'extrémité (3) et un second raccord d'extrémité (4) pouvant être raccordés, respectivement, à un premier tube (5) et à un second tube (6) pour soumettre un fluide de travail à travers l'élément tubulaire lui-même, et une portion intermédiaire (7) s'étendant entre ledit premier raccord d'extrémité (3) et ledit second raccord d'extrémité (4) et approprié pour soumettre le fluide à une mesure non invasive d'un ou de plusieurs paramètres,
dans lequel ledit élément tubulaire (2) comprend au moins deux pièces distinctes l'une de l'autre, dont une première portion tubulaire (2a) et au moins une deuxième portion (2b) portant un élément en saillie (8) approprié pour être inséré dans un renfoncement d'accouplement correspondant prévu sur un appareil médical pour activer la mesure optique, où ladite seconde portion (2b) est appropriée pour coopérer avec ladite première portion tubulaire (2a) pour fixer la position dudit élément en saillie (8) par rapport à la première portion tubulaire elle-même, **caractérisé en ce que**
ladite première portion tubulaire (2a) définit à la fois lesdits premier et second raccords d'extrémité (3, 4) et que ladite seconde portion (2b) est mobile par rapport à la première portion tubulaire elle-même et est appliquée sur la première portion tubulaire (2a) et le second tube (6) ajusté sur celle-ci ;
ou **en ce que**
ladite première portion tubulaire (2a) est du type rigide et que ledit élément en saillie (8) est réalisé avec un matériau souple.

2. Dispositif (1) selon la revendication 1, **caractérisé en ce que** ledit élément en saillie (8) est convexe et arrondi.

3. Dispositif (1) selon la revendication 1 ou 2, **caractérisé en ce que** ladite seconde portion (2b) comprend au moins un élément de base du type rigide et au moins un élément couvrant appliqué sur ledit élément de base, définissant ledit élément en saillie (8) et réalisé avec un matériau souple.

4. Dispositif (1) selon une ou plusieurs des revendications précédentes, **caractérisé en ce que** ladite première portion tubulaire (2a) et ladite seconde portion (2b) définissent ledit premier raccord d'extrémité (3) et ledit second raccord d'extrémité (4), respectivement, ladite seconde portion (2b) étant du type tubulaire, et **en ce que** ladite première portion tubulaire (2a) et ladite seconde portion (2b) sont fixées l'une à l'autre.

5. Dispositif (1) selon une ou plusieurs des revendications 1 à 3, **caractérisé en ce que** ladite seconde portion (2b) est mobile en translation et/ou en rotation par rapport à ladite première portion tubulaire (2a) le long et/ou autour, respectivement, de l'axe longitudinal de la première portion tubulaire elle-même.

6. Dispositif (1) selon la revendication 5, **caractérisé en ce que** ladite seconde portion (2b) comprend des moyens d'ancrage (9) de ladite seconde portion (2b) à ladite première portion tubulaire (2a), où ledit moyen d'ancrage (9) est raccordé de manière coulissante et/ou rotative à ladite première portion tubulaire (2a) par interposition du second tube (6).

7. Dispositif (1) selon la revendication 5 ou 6, **caractérisé en ce que** ladite seconde portion (2b) est en forme de pince ou annulaire.

8. Dispositif (1) selon une ou plusieurs des revendications 5 à 7, **caractérisé en ce que** ladite première portion tubulaire (2a) présente au moins une surface d'arrêt (10) définissant la course de fin de ladite seconde portion (2b) durant son mouvement le long de la direction longitudinale de la première portion tubulaire elle-même.

9. Dispositif (1) selon la revendication 8, **caractérisé en ce que** ladite surface d'arrêt (10) fait saillie radialement vers l'extérieur.

10. Dispositif (1) selon une ou plusieurs des revendications 5 à 9, **caractérisé en ce que** la section transversale dudit premier raccord d'extrémité (3) présente au moins une surface plate externe (11) adaptée à être placée, durant l'utilisation, face à l'appareil médical et à définir la position angulaire dudit élément en saillie (8).
